# EUROPEAN PATENT SPECIFICATION

(11) **EP 4 403 124 B1**
(45) Date of publication and mention of the grant of the patent: **18.11.2026**
(21) Application number: 22866804.2
(22) Date of filing: 13.09.2022
(51) Int. Cl.: A61B 18/12, A61B 18/14, A61M 25/00, A61B 18/00

(54) **ABLATION CATHETER**
ABLATIONSKATHETER
CATHÉTER D'ABLATION

(30) Priority: 13.09.2021 CN 202111067333
(43) Date of publication of application: 24.07.2024
(73) Proprietor: APT Medical Inc., Xiangtan, Hunan 410000 (CN)
(72) Inventor: XIE, Min, Xiangtan, Hunan 410000 (CN); ZHOU, Tuo, Xiangtan, Hunan 410000 (CN); CAO, Zhencai, Xiangtan, Hunan 410000 (CN); YANG, Yang, Xiangtan, Hunan 410000 (CN); ZHANG, Xiaokai, Xiangtan, Hunan 410000 (CN)
(74) Representative: Barker Brettell LLP
(86) International application number: PCT/CN2022/118507
(87) International publication number: WO 2023/036343

(56) References cited:
- EP-B1- 2 709 517
- WO-A1-2021/001772
- CN-A- 106 413 537
- CN-A- 112 842 518
- CN-A- 113 576 655
- US-A- 5 545 161
- US-A1- 2005 060 885
- US-B2- 7 896 873

## Description

### Technical Field

The present disclosure relates to the field of medical devices, and in particular, to an ablation catheter.

### Background

Atrial fibrillation is a type of tachyarrhythmia, and as the age increases, the incidence rate of atrial fibrillation increases continuously, and for people of 75 years of old or older, the incidence rate of atrial fibrillation can reach 10%. When atrial fibrillation occurs, the frequency of atrial activation reaches 300 - 600 times/minute, the ventricular rate is often fast and irregular, and sometimes can reach 100 - 160 times/minute, which is not only much faster than the heartbeat rhythm of a normal person, but also is absolutely irregular, and thus the atria lose an effective systole function. The prevalence rate of atrial fibrillation is also closely related to diseases such as coronary heart disease, hypertension and heart failure.

A distal structure of a conventional radio-frequency ablation catheter generally comprises a distal electrode, a ring electrode, and a catheter, etc. A typical internal structure thereof is that the distal electrode is fixed on a distal end of a catheter formed by an insulated material, and the ring electrode is attached to a catheter body of the catheter distal end. Electrode leads, which are welded on the ring electrode and the distal electrode, enter a catheter cavity of the catheter by through-holes on the catheter body, and are led out to an electrical connector at a proximal end of a handle through a proximal end of the catheter; and in such a catheter, only the distal electrode is used for transmitting ablation energy. A discharge loop is composed of an ablation energy generator, a distal electrode, a human body and a back plate; the ring electrode is only used for recording an intracardiac electric signal, which renders that each ablation can only be performed with regard to one point; this ablation manner not only has a low efficiency and is time-consuming, but also easily forms discontinuity of ablation lines (that is, when ablation is performed on one surface, because in conventional ablation, only the distal electrode participates in the ablation, there may be a certain point on this surface where ablation is not achieved). In addition, different electrodes and electrode leads easily interfere with one another, and cannot work normally, in particular under high-voltage ablation energy.

CN 112842518, US 5545161A, US 7896873B2, and EP 2709517 B1 all disclose background art.

### Summary

The scope of the invention is set out in the appended claims. An object of the present invention is to provide an ablation catheter, and the technical problem to be solved is the insulation problem during high-voltage discharge, so that a single electrode or a plurality of electrodes can participate in high-voltage discharge individually or simultaneously, thereby increasing the ablation efficiency.

In order to solve the technical problem, embodiments of the present disclosure adopt the following technical solutions:
embodiments of the present disclosure provide an ablation catheter, comprising: a catheter, a handle and a connector which are connected in sequence, wherein a plurality of electrodes are provided in sequence at a distal end of the catheter, each of the electrodes has a corresponding electrode lead, which passes through the catheter and the handle and is connected to the connector; the catheter comprises an insulated inner catheter, the electrodes are provided on the insulated inner catheter, an insulated outer catheter is provided between the plurality of electrodes, electrode leads of the plurality of electrodes are respectively provided between the insulated inner catheter and the insulated outer catheter.

In some embodiments, the distances between adjacent electrodes are equal.

In some embodiments, an adhesive is provided between the electrode leads and the insulated inner catheter to fix the positions of the electrode leads.

In some embodiments, first insulated sleeves are sleeved on each of the electrode leads, and the first insulated sleeves are provided between the insulated inner catheter and the insulated outer catheter.

In some embodiments, a sensor is provided in a catheter body at the distal end of the catheter, the sensor is a pressure sensor and/or a magnetic positioning sensor, and leads of the pressure sensor and/or the magnetic positioning sensor are provided between the insulated inner catheter and the insulated outer catheter.

In some embodiments, an insulated sleeve is sleeved on leads of the pressure sensor and/or the magnetic positioning sensor, and the second insulated sleeve is provided between the insulated inner catheter and the insulated outer catheter.

In some embodiments, the plurality of electrodes can simultaneously or individually release ablation energy, to perform ablation operations.

In some embodiments, the electrodes are distal electrodes or ring electrodes, each distal electrode is provided at a distal end of the catheter, and each ring electrode is provided around the catheter body of the catheter at other position except the distal end.

In some embodiments, a flexible section is provided at the distal end of the catheter, the flexible section can bend relative to the catheter, and the electrodes are provided at a catheter body portion where the flexible section is located.

In some embodiments, the flexible section comprises an annular body formed by winding a memory metal, and a flexible material is coated on the annular body in an annular shape or a spiral shape, or the flexible material is sleeved on the annular body directly.

In the embodiments of the present disclosure, the electrode leads of the plurality of electrodes are provided between different positions on the catheter body of the catheter, to ensure the insulation between the electrode leads and the insulation between different electrodes, and also enable different electrodes to apply high-voltage ablation energy individually or simultaneously, so that the plurality of electrodes participate in the transfer of ablation energy individually or simultaneously, thereby increasing the ablation efficiency.

### Brief Description of the Drawings

In order to describe the technical solutions in the embodiments of the present disclosure or in the related art more clearly, hereinafter, accompanying drawings requiring to be used for describing the embodiments or the related art are introduced briefly. Apparently, the accompanying drawings in the following description merely relate to some embodiments disclosed in the present disclosure, and for a person of ordinary skill in the art, other accompanying drawings can also be obtained according to these accompanying drawings without involving any inventive effort.
Fig. 1 is a schematic structural diagram of an ablation catheter according to embodiments of the present disclosure;
Fig. 2 is a schematic structural diagram of another distal end of an ablation catheter according to embodiments of the present disclosure;
Fig. 3 is a schematic structural diagram of another distal end of an ablation catheter according to embodiments of the present disclosure;
Fig. 4 is a schematic structural diagram of electrode leads of an ablation catheter according to embodiments of the present disclosure;
Fig. 5 is a schematic structural diagram of electrode arrangement of an ablation catheter according to embodiments of the present disclosure;
Fig. 6 is a schematic diagram of an ablation effect of electrode arrangement of an ablation catheter according to embodiments of the present disclosure;
Fig. 7 is a schematic structural diagram of electrode arrangement of an ablation catheter according to embodiments of the present disclosure;
Fig. 8 is a schematic diagram of an ablation effect of electrode arrangement of an ablation catheter according to embodiments of the present disclosure;
Fig. 9 is a schematic structural diagram of electrode arrangement of an ablation catheter according to embodiments of the present disclosure;
Fig. 10 is a schematic diagram of an ablation effect of electrode arrangement of an ablation catheter according to embodiments of the present disclosure;
Fig. 11 is a schematic diagram of arrangement of electrode leads in an ablation catheter according to embodiments of the present disclosure;
Fig. 12 is a schematic diagram of arrangement of electrode leads in an ablation catheter according to embodiments of the present disclosure;
Fig. 13 is a schematic diagram of arrangement of electrode leads in an ablation catheter according to embodiments of the present disclosure; and
Fig. 14 is a schematic diagram of arrangement of sensors in an ablation catheter according to embodiments of the present disclosure.

### Reference signs:

1 - Catheter; 2 - Distal electrode; 21 - First electrode lead; 3 - Ring electrode; 31 - Second electrode lead; 4 - Pressure sensor; 5 - Magnetic positioning sensor; 6 - Second insulated sleeve; 7 - Connector; 8 - First insulated sleeve; 9 - Handle; 11 - Flexible section; 12 - Insulated inner catheter; 13 - Insulated outer catheter; 100 - Ablation catheter.

### Detailed Description of the Embodiments

Various solutions and features of the present disclosure are described herein with reference to the accompanying drawings.

It will be appreciated that various modifications can be made to the embodiments of the application herein. Accordingly, the description should not be construed as limiting but merely as exemplifications of the embodiments. A person skilled in the art would have conceived of other modifications within the scope and spirit of the present disclosure.

The accompanying drawings, which are incorporated in the description and constitute a part of the description, illustrate embodiments of the present disclosure, and together with a general description of the present disclosure given above and the detailed description of the embodiments given below, serve to explain the principle of the present disclosure.

These and other features of the present disclosure will become apparent from the following description of preferred forms of embodiments given as non-limiting examples with reference to the accompanying drawings.

It should also be understood that while the present disclosure has been described with reference to specific examples, a person skilled in the art would be able to conclusively implement many other equivalent forms of the present disclosure having the features of the claims and thus all being within the scope of protection defined thereby.

The above and other aspects, features, and advantages of the present disclosure will become more apparent in view of the following detailed description when taken in conjunction with the accompanying drawings.

Hereinafter, specific embodiments of the present disclosure are described with reference to the accompanying drawings. However, it should be understood that the embodiments as applied are merely examples of the present disclosure, which may be implemented in various ways. Well-known and/or duplicate functions and structures have not been described in detail to avoid unnecessary or redundant details which obscure the present disclosure. Therefore, specific structural and functional details as applied herein are not intended for limiting, but merely as a basis and a representative basis for the claims and for teaching a person skilled in the art to diversely use the present disclosure in substantially any suitable detailed structure.

The description may use phrases "in one embodiment", "in another embodiment", "in yet another embodiment", or "in other embodiments", which may each refer to one or more of the same or different embodiments according to the present disclosure.

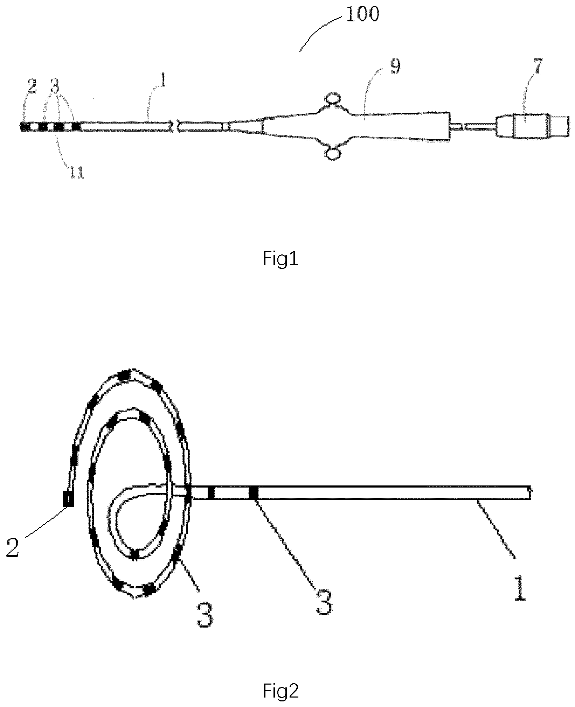

A first embodiment of the present disclosure provides an ablation catheter. The ablation catheter can achieve an ablation operation at positions such as myocardium in a patient's body, under the condition of high-voltage ablation energy provided from the outside. As shown in Fig. 1, the ablation catheter 100 comprises a catheter 1, a handle 9 and a connector 7 which are connected in sequence, wherein the catheter 1 is located at a distal end of the ablation catheter 100, and can extend into operating positions, such asmyocardium, etc. in a patient's body during a surgery, so as to perform an ablation operation on a lesion; and the connector 7 is located at a proximal end of the ablation catheter 100, and is used for connecting to an external ablation energy generator. The ablation energy herein is used for achieving an ablation operation at a designated position, and an operator can control a specific ablation operation by the handle 9. For example, the curvature of the distal end of the catheter 1 can be adjusted by the handle 9, and the handle 9 can also start or close an ablation operation, or adjust the intensity value of the ablation energy, etc. In addition, the distal end herein refers to the end away from the operator, and the proximal end refers to the end near the operator.

Furthermore, with continued reference to Fig. 1, in an optional embodiment, a flexible section 11 is provided at the distal end of the catheter 1, the flexible section 11 can bend relative to the catheter 1, so as to facilitate adjustment of the curvature in the patient's body according to different operating positions. Generally, the flexible section can be made of a material easy to bend, so that the rigidity of the catheter body portion, of the catheter 1, other than the flexible section 11 is greater than the rigidity of the catheter body where the flexible section 11 is located, and thus after the catheter 1 extends into the patient's body, the catheter body at the distal end of the catheter 1 can be twisted, thereby being able to adjust the bending direction of the flexible section 11 according to the operating position.

Furthermore, considering that the flexible section 11 may be made of a material easy to bend, but may lack a certain degree of rigidity to stabilize the bending state, the curvature of the flexible section 11 needs to be recalibrated each time. According to that, the structure of the flexible section 11 can be improved. In one embodiment, an annular body formed by winding memory metal is provided at the distal end of the catheter 1, and a flexible material is coated on the annular body in a free state in an annular shape or a spiral shape. In this way, the distal end of the catheter 1 can be deformed under an external force, and can be restored in a free state. In a manufacturing process, the flexible material can form the flexible section on the annular body by means of hot melting, or the flexible material can be sleeved on the annular body directly to form the flexible section.

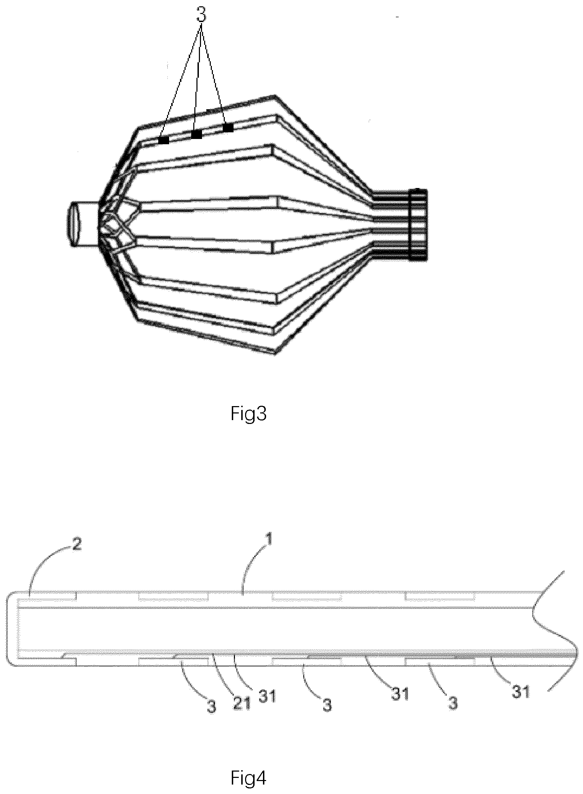

In order to adapt to different usage environments, the distal end of the catheter 1 may be performed in various forms, for example, as shown in Fig. 1, the distal end of the catheter 1 herein may be of a linear type; as shown in Fig. 2, the distal end of the catheter 1 may also be a spiral type, and certainly, may also be set as a mesh basket type as shown in Fig. 3.

Additionally, a plurality of electrodes are provided at the distal end of the catheter 1, each of the electrodes has a corresponding electrode lead, which passes through the catheter 1 and the handle 9 and is finally connected to the connector 7. Here, the electrodes may be, for example, distal electrodes or ring electrodes, depending on the different arrangement position.

Taking Fig. 1 as an example, in the structure of the catheter 1 which has a linear distal end as shown in Fig. 1, a distal electrode 2 is provided at a distal end of the catheter 1, and the distal electrode 2 can perform an ablation operation on a lesion near the distal end position of the catheter 1. At least one ring electrode 3 is provided around the catheter body of the catheter 1 at other position except the distal end, the ring electrode 3 may be, for example, sleeved on the catheter body of the catheter 1, and the ring electrode 3 can perform an ablation operation on a lesion at a position close to a side surface of the catheter 1.

Taking Fig. 2 as an example, in the structure of the catheter 1 which has a spiral distal end as shown in Fig. 2, a distal electrode 2 is provided at a distal end of the catheter 1, and the distal electrode 2 can perform an ablation operation on a lesion near the distal end position of the catheter 1. At least one ring electrode 3 is provided around the catheter body of the catheter 1 except the spiral catheter body at the distal end, the ring electrode 3 may be, for example, sleeved on the catheter body of the catheter 1, and the ring electrode 3 can perform an ablation operation on a lesion at a position close to a side surface of the catheter 1. Here, for the spiral distal end, if there are shaping requirements, a material having adjustable curvature may be used to form the spiral shape, or a metal wire is added in the inner cavity of the catheter body to set certain curvature.

Taking Fig. 3 as an example, in the structure of the catheter 1 which has a mesh basket-type distal end as shown in Fig. 3, the mesh basket-type distal end comprises a plurality of arms; at least one ring electrode 3 is provided around each arm of the catheter body of the catheter 1, the ring electrode 3 may be, for example, sleeved on the arm, and the ring electrode 3 can perform an ablation operation on a lesion at a position close to a side surface of the arm.

In addition, in another embodiment, on the catheter 1 provided with the flexible section 11, the distal electrode 2 and/or the ring electrode 3 are preferably provided on a catheter body portion where the flexible section 11 is located, thereby facilitating combination with the bending characteristic of the flexible section 11, so as to achieve an ablation operation at some bending positions in the patient's body, but embodiments of the present disclosure are not limited thereto. Certainly, the ring electrode 3 may also be provided on the remaining catheter body portion other than the flexible section 11 on the catheter 1.

In one embodiment, in order to extend the area involved in an ablation operation, a plurality of ring electrodes 3 may be provided on the catheter 1. For example, the plurality of ring electrodes 3 can be provided on the catheter body of the linear distal end, the catheter body of the spiral distal end, or the arms of the mesh basket-type distal end; and when multiple ring electrodes 3 are applied, the plurality of ring electrodes 3 are provided on the catheter 1 in sequence at intervals.

Furthermore, in different forms of the catheter 1, the distance between the distal electrode 2 and an adjacent ring electrode 3 or the distance between adjacent ring electrodes 3 may be equal, and thus it can be ensured that the damage characteristics between different electrodes are similar in an ablation operation process, so as to avoid the situation of discontinuity of damage between certain two electrodes. Here, the distance may be set according to the length of the catheter 1 or actual operation requirements, which is not specifically limited herein.

Furthermore, the lengths of the ring electrodes 3 along the length direction of the catheter 1 may also be set to be equal. In this way, it can be ensured that the ring electrodes 3 can achieve uniform output of ablation energy, so that damage regions formed by the ring electrodes 3 have substantially the same size, thereby avoiding a problem of insufficient ablation or excessive ablation at a local part.

Considering that each of the electrodes has a corresponding electrode lead, as shown in Fig. 4, in the catheter 1 which has a linear distal end, the distal electrode 2 has a first electrode lead 21, and each of the ring electrodes 3 has a second electrode lead 31. Here, the first electrode lead 21 and the second electrode leads 31 are respectively led out from the distal electrode 2 and the ring electrodes 3, and accommodated in the catheter 1. Here, the first electrode lead 21 and the second electrode leads 31 pass through the catheter body of the catheter 1 and the handle 9, and finally, are electrically connected to the connector 7 at the proximal end of the handle 9, so as to send ablation energy to the distal electrode 2 and the ring electrodes 3 by an external electric signal generator.

Certainly, each of the distal electrode 2 or the ring electrodes 3 on the catheter 1 of the spiral distal end and the catheter 1 of the mesh basket-type distal end has a corresponding electrode lead, which are not repeated herein. Hereinafter, how to control different electrodes independently and achieve insulation between different electrodes will be introduced by taking the catheter 1 of the linear distal end as an example.

Furthermore, the different electrodes provided at the distal end of the catheter 1 can simultaneously or individually release ablation energy to perform ablation operations, and high-voltage insulation needs to be achieved between the different electrodes and electrode leads.

For example, in the catheter 1 having a linear distal end, in particular in the catheter 1 having a linear distal end, as shown in Fig. 4, considering that the first electrode lead 21 of the distal electrode 2 and the second electrode leads 31 of the ring electrodes 3 are provided in the catheter body of the catheter 1, mutual insulation needs to be achieved between the distal electrode 2 and the ring electrodes 3 and the first electrode lead 21 and the second electrode leads 31, so as to ensure that there is no interference between each other, thereby achieving the effect of insulation and high-voltage resistance. In this way, high-voltage ablation energy can be applied between the plurality of electrodes, so that the plurality of different electrodes simultaneously participate in the transfer of ablation energy, finally increasing the ablation efficiency for a lesion.

Here, the distal electrode 2 and the ring electrodes 3 may simultaneously or individually release ablation energy to perform ablation operations , and the ablation energy herein may be radio frequency signals, and may also be pulsed electric field signals. The type of the ablation energy is not specifically limited herein, and it is only necessary to ensure that the released ablation energy can perform a lesion in an ablation operation at a specified location.

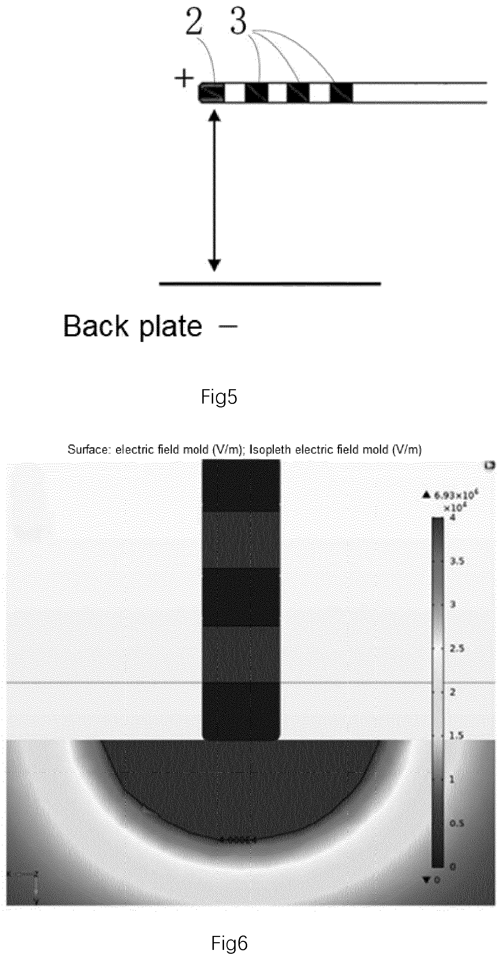

Specifically, as shown in Figs. 5 and 6, when only the distal electrode 2 releases an electric signal, unipolar ablation of discharge is performed between the distal electrode and a back plate. Thus, a unipolar ablation mode of discharge between the distal electrode 2 and the back plate is used; in this manner, ablation on a damaged region is in point contact, and the ablation range is small.

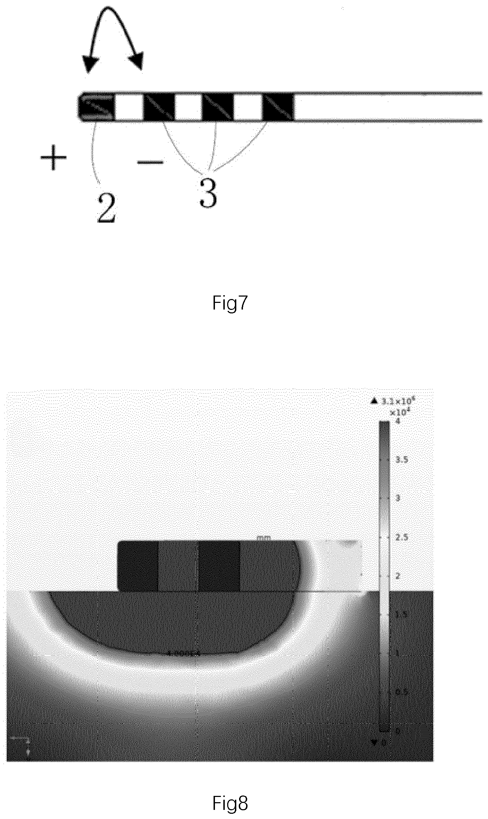

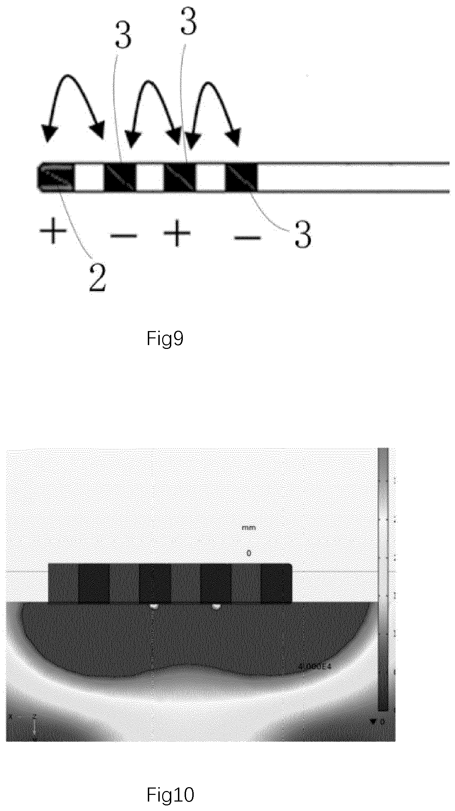

As shown in Figs. 7-10, when the distal electrode 2 and the ring electrodes 3 release electric signals at the same time, the polarity of the distal electrode 2 is positive, and the polarities of all other ring electrodes 3 are negative; or the polarity of at least one ring electrode 3 is positive, and the polarities of the remaining electrodes are all negative. According to that, it is only necessary to ensure that at least positive polarity and negative polarity both participate in the release of electric signals where the distal electrode 2 and the ring electrodes 3 release electric signals at the same time. For example, the polarity arrangement sequence of the distal electrode 2 and the plurality of ring electrodes 3 may be +---+, +-+-+- or -+---etc., which are not defined in the embodiments of the present disclosure. Specifically, in an embodiment, as shown in Figs. 7 and 8, which show that a bipolar discharge mode is achieved by the distal electrode 2 and a ring electrode 3 adjacent thereto, the damage range of the bipolar discharge mode in the long axis direction of the catheter 1 is wider, so that the ablation range is larger than that in the unipolar ablation mode. Furthermore, as shown in Figs. 9 and 10, which show another bipolar discharge mode of the distal electrode 2 and a ring electrode 3 adjacent thereto, the damage range of this bipolar discharge mode in the long axis direction of the catheter 1 is wider, and thus the ablation range is also larger than that in the unipolar ablation mode. In this way, in the embodiments of the present disclosure, in a process in which a plurality of electrodes simultaneously participate in discharge, a linear continuous ablation band can be formed at one time, thereby avoiding the problem that when single-point ablation is performed, actual ablation points deviate from a predetermined ablation line, so that the ablation points cannot be continuous to form a line and the ablation points are discontinuous.

The bipolar discharge ablation according to the embodiments of the present disclosure can also restrict ablation energy to a peripheral region of the electrodes, so as to avoid a situation in a traditional unipolar ablation mode in which ablation energy flows through a body and then is collected on the back plate, that is, to avoid side effects such as nerve stimulation (pain), muscle contraction, and burn on the back plate which easily occur in this process, thereby improving the safety of a surgery.

Therefore, by using the unipolar and bipolar discharge control modes of the distal electrode 2 and/or the ring electrodes 3, the requirements for ablation in various complex surgeries such as tricuspid valve linear ablation, left atrial top line, left atrial bottom line, and mitral valve isthmic line in arrhythmia therapy can be satisfied, and the surgery efficiency and success rate can be increased.

Furthermore, as described above, in consideration of the need to achieve mutual insulation between the distal electrode 2 and the ring electrodes 3 and between the first electrode lead 21 and the second electrode leads 31, in for example, the catheter 1 having a linear distal end, thereby achieving the effect of insulation and high-voltage resistance, the catheter 1 can be made of a high-polymer insulator material, so as to ensure the insulation effect between the welding spots of the electrode leads of different electrodes and the electrode leads. As the electric signals are high-voltage signals, the electrode leads of the plurality of electrodes are insulated from each other to avoid short-circuiting.

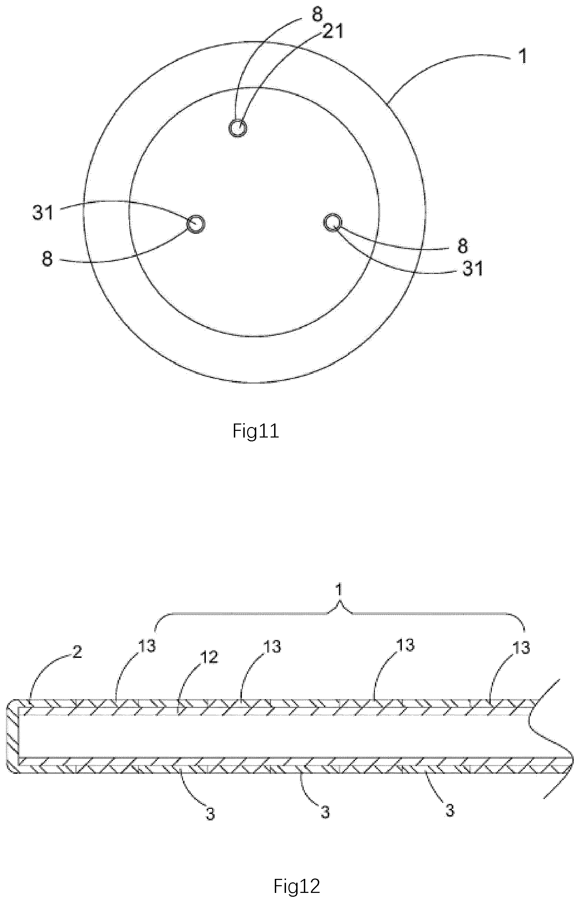

In order to achieve insulation between the electrode leads of different electrodes, for example, in order to achieve insulation between the first electrode lead 21 and the second electrode leads 31, as shown in Fig. 11, first insulated sleeves 8 can be sleeved on the first electrode lead 21 of the distal electrode 2 and the second electrode leads 31 of the ring electrodes 3, and thus the electrode leads are provided on the first insulated sleeves 8 to achieve insulation, for example, by hot melting, the first insulated sleeves 8 can be fixed on corresponding electrode leads, thereby achieving a better insulation effect.

Certainly, in order to relatively fix the positions of the first insulated sleeves 8, so that different first insulated sleeves 8 are spaced apart from one another at certain distances, an adhesive can be provided on the first ieleeve nsulated sleeves 8 to fix the first insulated sleeves on the inner wall of the catheter body of the catheter 1 at predetermined positions. Different predetermined positions herein may be spaced apart from one another at certain distances, so that different first insulated sleeves 8 are spaced apart from one another at certain distances.

In addition, in order to improve the insulation effect between different electrode leads, the structure of the catheter 1 can be improved. In one embodiment, the catheter 1 may be constructed by using the following as shown in Fig. 12, the catheter 1 comprises an insulated inner catheter 12, and the distal electrode 2 is provided at a distal end of the insulated inner catheter 12, at least one ring electrode 3 is sleeved on the catheter body of the insulated inner catheter 12, and an insulated outer catheter 13 is provided between the distal electrode 2 and each ring electrode 3. Here, both the insulated inner catheter 12 and the insulated outer catheter 13 can be made of plastic. An end of the insulated outer catheter 13 is connected to the distal electrode 2, and the outer wall of each ring electrode 3 is flush with the outer wall of the catheter body of the insulated outer catheter 13. Here, the first electrode lead 21 of the distal electrode 2 and the second electrode lead 31 of the ring electrode 3 are provided between the insulated inner catheter 12 and the insulated outer catheter 13, and the positions thereof are fixed by pressing between the insulated inner catheter 12 and the insulated outer catheter 13. Certainly, an adhesive may be provided between the electrode leads and the insulated inner catheter 12 to fix the positions of the electrode leads on the insulated inner catheter 12. In this way, the catheter 1 uses two layers of plastic insulation catheters and electrode leads corresponding to different electrodes are fixed in the middle of the catheter, so as to achieve the advantages of insulation and non-breakdown of the catheter.

Here, the insulated outer catheter 13 may partially extend to the position of the distal electrode 2, so as to partially wrap the distal electrode 2 to form fixation. Certainly, the distal electrode 2 may also be connected to the insulated outer catheter 13.

In this way, the first electrode lead 21 of the distal electrode 2 and the second electrode lead 31 of the ring electrode 3 are distributed in the radial direction of the catheter body of the catheter 1 and are staggered from each other. Since the voltage of pulse discharge can reach one thousand volts, the electrode leads of different electrodes being staggered from each other can prevent the electrode leads from being broken down, thereby having better high-voltage resistance.

In a manufacturing process, the ring electrode 3 is knocked by a mold pressing process at mechanical rapid 360-degree rotation, so that the outer diameter of the ring electrode 3 is uniformly reduced, thereby tightly pressing the ring electrode onto the insulated inner catheter 12. However, the embodiments of the present disclosure are not limited thereto, and it may also be achieved in a manner of tightly combining a metal annular object and a plastic catheter in the related art, which is not specifically limited herein. Furthermore, the insulated outer catheter 13 is hot-melted and fixed on the surface of the insulated inner catheter 12 in a hot-melting process.

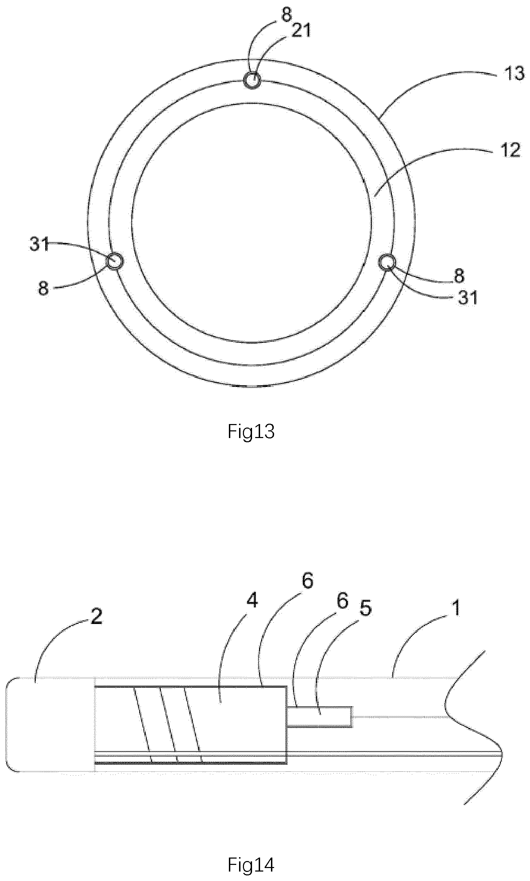

Based on above, as shown in Fig. 13, first insulated sleeves 8 can be sleeved on the first electrode lead 21 of the distal electrode 2 and the second electrode lead 31 of the ring electrode 3, and the first insulated sleeves 8 are provided in an annular region between the insulated inner catheter 12 and the insulated outer catheter 13, such that different first insulated sleeves 8 are arranged at intervals in the annular region, thereby further improving the insulation effect. Compared with fixing the electrode leads between the insulated inner catheter 12 and the insulated outer catheter 13, fixing the first insulated sleeves 8 with a larger radius between the insulated inner catheter 12 and the insulated outer catheter 13 can enable the positions of the electrode leads to be fixed more firmly, and prevent the positions of the electrode leads from moving during use, thereby maintaining the insulation effect. Here, it can be ensured that the first insulated sleeves 8 are arranged separately, so as to ensure that there is a certain distance between different first insulated sleeves 8, thereby preventing the electrode leads from interfering with each other.

Furthermore, in some other embodiments, as shown in Fig. 14, after the ablation catheter 1 extends into the position of the myocardium in the patient's body, in order to acquire information such as the degree of attachment between the distal end of the catheter 1 and the myocardium in the body and the position of the distal end of the catheter 1, etc., a sensor can be further provided in the catheter body at the distal end of the catheter 1, for example, a pressure sensor 4 and/or a magnetic positioning sensor 5 are provided. Specifically, the pressure sensor 4 and/or the magnetic positioning sensor 5 are arranged adjacent to the distal electrode 2, and the leads thereof also extend through the catheter cavity of the catheter 1 and extends out of the proximal end of the catheter 1.

In order to ensure the insulation requirements between the leads of the sensors and the electrode leads, second insulated sleeves 6 are coated on the outer sides of the pressure sensor 4 and/or the magnetic positioning sensor 5, and the proximal end of each second insulated sleeve 6 is sealed with an adhesive. The leads of the pressure sensor 4 and/or the magnetic positioning sensor 5 may be provided in the catheter body of the catheter 1, for example, arranged between the insulated inner catheter 12 and the insulated outer catheter 13 or directly fixed between the insulated inner catheter 12 and the insulated outer catheter 13 by the second insulated sleeves 6, to achieve the purpose of insulation. The second insulated sleeves 6 herein are made of high-polymer insulator materials. During provision, the leads of the pressure sensor 4 and/or the magnetic positioning sensor 5 or the second insulated sleeves 6 sleeved outside the leads, and the electrode leads, and the first insulated sleeves 8 sleeved outside the electrode leads are spaced apart in advance and the positions thereof are fixed, so as to satisfy the insulation requirements between different leads.

In the embodiments of the present disclosure, the electrode leads of the plurality of electrodes are provided between different positions on the catheter body of the catheter, to ensure the insulation between the electrode leads and the insulation between different electrodes, and also enable different electrodes to apply high-voltage ablation energy individually or simultaneously, so that the plurality of electrodes participate in the transfer of ablation energy individually or simultaneously, thereby increasing the ablation efficiency.

In addition, although various operations are depicted in a specific order, this should not be understood as requiring that these operations be performed in the specific order shown or in sequential order. In certain environments, multi-task and parallel processing may be advantageous. Likewise, while several specific implementation details are included in the discussion above, these should not be construed as limiting the scope of the present disclosure. Certain features that are described in the context of individual embodiments can also be implemented in a single embodiment in a combination manner. Conversely, various features that are described in the context of a single embodiment can also be implemented in multiple embodiments individually or in any suitable subcombination manner.

Although the subject matter has been described in language specific to structural features and/or methodological logic acts, it should be understood that the subject matter defined in the appended claims is not necessarily limited to the specific features or acts described above. Rather, the specific features and acts described above are merely exemplary forms for implementing the claims.

## Claims

1. An ablation catheter, comprising: a catheter (1), a handle (9) and a connector (7) which are connected in sequence, wherein a plurality of electrodes are provided in sequence at a distal end of the catheter (1), each of the electrodes has a corresponding electrode lead, which passes through the catheter (1) and the handle (9) and is connected to the connector (7); **characterized in that** the catheter (1) comprises an insulated inner catheter (12), the electrodes are provided on the insulated inner catheter (12), an insulated outer catheter (13) is provided between the plurality of electrodes, wherein the electrode leads of the plurality of electrodes are respectively provided between the insulated inner catheter (12) and the insulated outer catheter (13) and the positions thereof are fixed by pressing between the insulated inner catheter (12) and the insulated outer catheter (13).

2. The ablation catheter according to claim 1, wherein the distances between adjacent electrodes are equal.

3. The ablation catheter according to claim 1, wherein an adhesive is provided between the electrode leads and the insulated inner catheter (12), to fix the positions of the electrode leads.

4. The ablation catheter according to claim 1, wherein first insulated sleeves (8) are sleeved on each of the electrode leads, and the first insulated sleeves (8) are provided between the insulated inner catheter (12) and the insulated outer catheter (13).

5. The ablation catheter according to claim 1, wherein a sensor is provided in a catheter body at the distal end of the catheter (1), the sensor is a pressure sensor (4) and/or a magnetic positioning sensor (5), and leads of the pressure sensor (4) and/or the magnetic positioning sensor (5) are provided between the insulated inner catheter (12) and the insulated outer catheter (13).

6. The ablation catheter according to claim 5, wherein a second insulated sleeves (6) are sleeved on leads of the pressure sensor (4) and/or the magnetic positioning sensor (5), and the second insulated sleeve (6) is provided between the insulated inner catheter (12) and the insulated outer catheter (13).

7. The ablation catheter according to claim 1, wherein the plurality of electrodes can simultaneously or individually release ablation energy, to perform ablation operations.

8. **The** ablation catheter according to claim 1, wherein the electrodes are distal electrodes (2) or ring electrodes (3), each distal electrode (2) is provided at a distal end of the catheter (1), and each ring electrode (3) is provided around the catheter body of the catheter (1) at other position except the distal end.

9. **The** ablation catheter according to claim 1, wherein a flexible section (11) is provided at the distal end of the catheter (1), the flexible section (11) can bend relative to the catheter (1), and the electrodes are provided at a catheter body portion where the flexible section (11) is located.

10. **The** ablation catheter according to claim 9, wherein the flexible section (11) comprises an annular body formed by winding memory metal, and a flexible material is coated on the annular body in an annular shape or a spiral shape, or the flexible material is sleeved on the annular body directly.

## Patentansprüche

1. Ablationskatheter, umfassend: einen Katheter (1), einen Griff (9) und einen Verbinder (7), die nacheinander verbunden sind, wobei eine Vielzahl von Elektroden nacheinander an einem distalen Ende des Katheters (1) bereitgestellt ist, jede der Elektroden eine entsprechende Elektrodenleitung aufweist, die durch den Katheter (1) und den Griff (9) hindurch verläuft und mit dem Verbinder (7) verbunden ist; **dadurch gekennzeichnet, dass** der Katheter (1) einen isolierten Innenkatheter (12) umfasst, die Elektroden auf dem isolierten Innenkatheter (12) bereitgestellt sind, ein isolierter Außenkatheter (13) zwischen der Vielzahl von Elektroden bereitgestellt ist, wobei die Elektrodenleitungen der Vielzahl von Elektroden jeweils zwischen dem isolierten Innenkatheter (12) und dem isolierten Außenkatheter (13) bereitgestellt sind und die Positionen davon durch Drücken zwischen dem isolierten Innenkatheter (12) und dem isolierten Außenkatheter (13) fixiert sind.

2. Ablationskatheter nach Anspruch 1, wobei die Abstände zwischen benachbarten Elektroden gleich sind.

3. Ablationskatheter nach Anspruch 1, wobei ein Haftstoff zwischen den Elektrodenleitungen und dem isolierten Innenkatheter (12) bereitgestellt ist, um die Positionen der Elektrodenleitungen zu fixieren.

4. Ablationskatheter nach Anspruch 1, wobei erste isolierte Mäntel (8) jede der Elektrodenleitungen ummanteln und die ersten isolierten Mäntel (8) zwischen dem isolierten Innenkatheter (12) und dem isolierten Außenkatheter (13) bereitgestellt sind.

5. Ablationskatheter nach Anspruch 1, wobei ein Sensor in einem Katheterkörper an dem distalen Ende des Katheters (1) bereitgestellt ist, der Sensor ein Drucksensor (4) und/oder ein magnetischer Positionierungssensor (5) ist und Leitungen des Drucksensors (4) und/oder des magnetischen Positionierungssensors (5) zwischen dem isolierten Innenkatheter (12) und dem isolierten Außenkatheter (13) bereitgestellt sind.

6. Ablationskatheter nach Anspruch 5, wobei eine zweite isolierte Mäntel (6) Leitungen des Drucksensors (4) und/oder des magnetischen Positionierungssensors (5) ummanteln und der zweite isolierte Mantel (6) zwischen dem isolierten Innenkatheter (12) und dem isolierten Außenkatheter (13) bereitgestellt ist.

7. Ablationskatheter nach Anspruch 1, wobei die Vielzahl von Elektroden gleichzeitig oder einzeln Ablationsenergie freisetzen kann, um Ablationsvorgänge durchzuführen.

8. Ablationskatheter nach Anspruch 1, wobei die Elektroden distale Elektroden (2) oder Ringelektroden (3) sind, jede distale Elektrode (2) an einem distalen Ende des Katheters (1) bereitgestellt ist und jede Ringelektrode (3) um den Katheterkörper des Katheters (1) an anderer Position außer dem distalen Ende bereitgestellt ist.

9. Ablationskatheter nach Anspruch 1, wobei ein flexibler Abschnitt (11) an dem distalen Ende des Katheters (1) bereitgestellt ist, sich der flexible Abschnitt (11) in Bezug auf den Katheter (1) biegen kann und die Elektroden an einem Katheterkörperabschnitt bereitgestellt sind, an dem sich der flexible Abschnitt (11) befindet.

10. Ablationskatheter nach Anspruch 9, wobei der flexible Abschnitt (11) einen ringförmigen Körper umfasst, der durch Wickeln von Gedächtnismetall gebildet ist, und der ringförmige Körper mit einem flexiblen Material in einer Ringform oder einer Spiralform beschichtet ist oder das flexible Material den ringförmigen Körper unmittelbar ummantelt.

## Revendications

1. Cathéter d'ablation, comprenant : un cathéter (1), une poignée (9) et un connecteur (7) qui sont connectés en séquence, dans lequel une pluralité d'électrodes sont prévues en séquence au niveau d'une extrémité distale du cathéter (1), chacune des électrodes a un fil d'électrode correspondant, qui passe à travers le cathéter (1) et la poignée (9) et est connecté au connecteur (7) ; **caractérisé en ce que** le cathéter (1) comprend un cathéter interne isolé (12), les électrodes sont prévues sur le cathéter interne isolé (12), un cathéter externe isolé (13) est prévu entre la pluralité d'électrodes, dans lequel les fils d'électrode de la pluralité d'électrodes sont respectivement prévus entre le cathéter interne isolé (12) et le cathéter externe isolé (13) et les positions de ceux-ci sont fixées par pression entre le cathéter interne isolé (12) et le cathéter externe isolé (13).

2. Cathéter d'ablation selon la revendication 1, dans lequel les distances entre des électrodes adjacentes sont égales.

3. Cathéter d'ablation selon la revendication 1, dans lequel un adhésif est prévu entre les fils d'électrode et le cathéter interne isolé (12), pour la fixation des positions des fils d'électrode.

4. Cathéter d'ablation selon la revendication 1, dans lequel des premiers manchons isolés (8) sont emmanchés sur chacun des fils d'électrode, et les premiers manchons isolés (8) sont prévus entre le cathéter interne isolé (12) et le cathéter externe isolé (13).

5. Cathéter d'ablation selon la revendication 1, dans lequel un capteur est prévu dans un corps de cathéter au niveau de l'extrémité distale du cathéter (1), le capteur est un capteur de pression (4) et/ou un capteur de positionnement magnétique (5), et des fils du capteur de pression (4) et/ou du capteur de positionnement magnétique (5) sont prévus entre le cathéter interne isolé (12) et le cathéter externe isolé (13).

6. Cathéter d'ablation selon la revendication 5, dans lequel a seconds manchons isolés (6) sont emmanchés sur des fils du capteur de pression (4) et/ou du capteur de positionnement magnétique (5), et le second manchon isolé (6) est prévu entre le cathéter interne isolé (12) et le cathéter externe isolé (13).

7. Cathéter d'ablation selon la revendication 1, dans lequel la pluralité d'électrodes peuvent libérer simultanément ou individuellement une énergie d'ablation, pour la réalisation d'opérations d'ablation.

8. Cathéter d'ablation selon la revendication 1, dans lequel les électrodes sont des électrodes distales (2) ou des électrodes annulaires (3), chaque électrode distale (2) est prévue au niveau d'une extrémité distale du cathéter (1), et chaque électrode annulaire (3) est prévue autour du corps de cathéter du cathéter (1) à une autre position à l'exception de l'extrémité distale.

9. Cathéter d'ablation selon la revendication 1, dans lequel une section flexible (11) est prévue au niveau de l'extrémité distale du cathéter (1), la section flexible (11) peut se plier par rapport au cathéter (1), et les électrodes sont prévues au niveau d'une portion de corps de cathéter où la section flexible (11) est située.

10. Cathéter d'ablation selon la revendication 9, dans lequel la section flexible (11) comprend un corps annulaire formé par l'enroulement d'un métal à mémoire de forme, et un matériau flexible est revêtu sur le corps annulaire selon une forme annulaire ou une forme en spirale, ou le matériau flexible est emmanché directement sur le corps annulaire.
